# EUROPEAN PATENT SPECIFICATION

(11) **EP 1 819 372 B1**
(45) Date of publication and mention of the grant of the patent: **16.12.2009**
(21) Application number: 05823528.4
(22) Date of filing: 02.12.2005
(51) Int. Cl.: A61L 27/30, A61L 27/54, A61F 2/00, B05D 7/02

(54) **PROCESS FOR THE PRODUCTION OF SILVER-CONTAINING PROSTHETIC DEVICES**
VERFAHREN ZUR HERSTELLUNG VON SILBERHALTIGEN PROTHESEN
PROCEDE DE PRODUCTION DE PROTHESES CONTENANT DE L'ARGENT

(30) Priority: 02.12.2004 IT TO20040854
(43) Date of publication of application: 22.08.2007
(73) Proprietor: POLITECNICO DI TORINO, 10129 Torino (IT)
(72) Inventor: DI NUNZIO, Serena, 10144 Torino (IT); VERNE', Enrica, I-10138 Torino (IT)
(74) Representative: Rambelli, Paolo
(86) International application number: PCT/EP2005/056391
(87) International publication number: WO 2006/058906

(56) References cited:
- WO-A-00/76486
- WO-A-02/18003
- WO-A-03/003831
- WO-A-03/089023
- WO-A-2004/101011
- WO-A-2005/087982
- SCHIERHOLZ J M ET AL: "Efficacy of silver-coated medical devices" 1998, JOURNAL OF HOSPITAL INFECTION, ACADEMIC PRESS, LONDON, GB, PAGE(S) 257-262 , XP002295856 ISSN: 0195-6701 the whole document

## Description

The present invention refers to a process for the production of prosthetic devices or medical implants with antibacterial action, due to silver ions release.

One of the most common post-operative complications rising as a consequence of prostheses insertion is represented by infections onset [1]. This kind of problem can be solved by systemic supply of antibiotics, or by the antibacterial agents' aid, as for example silver ions, that being released gradually and in a controlled way directly on the implant site, can defeat the colonization by most common bacteria stocks of the surfaces of the implanted devices [2-8].

In respect of the commonly used antibiotics, silver has the advantage to be antibacterial in a wide range, can be supplied locally rather than orally, thus avoiding common antibiotics' side effects (as for example the development of resistant bacteria populations, intestinal flora impoverishment...). Polymeric devices coated with silver or containing silver ions and having antibacterial behaviour [9-13] are already commercialized (catheters, dressings for burn wound healing, toothbrushes (Silvercare®), filtering and purifying systems for drinking water or swimming pools, textiles fibres (X-Static®, Eastlon®, Amicor©) to realize antibacterial (Atisorb®) and anti-fungi tissues, plasters).

Those products are successfully used, but for devices that are not designed to be implanted, or for devices that come in contact with human body only for short time periods (disposable).

Some studies to produce implantable devices, suitable for silver ions release, have been performed in the field of metallic materials for orthopaedics (pins for external bone-fracture fixation devices) [14-17]; the only clinical tests, performed on steel orthopaedic screws, subjected to silver ion implantation [17], have not led to the routine application of the studied devices because it was impossible to dose, with the required accuracy, the contained silver, thus producing toxic effects on surrounding tissues and silver concentrations higher than allowed level for human being.

Studies in the field of glass, ceramic and glass-ceramic materials are actually in progress [18-22]. These studies generally regard synthesis techniques which modify the composition of all material body with the disadvantages above mentioned. Such obtained materials demonstrated an expected antibacterial activity, but they have never been used or tested clinically, nor is their cytotoxic behaviour known.

Preliminary studies of ion exchange treatments in molten salts mixtures, performed by the research group of the inventors of the present patent [32] have demonstrated the possibility to enrich with silver ions the biomaterial surface only, but reaching a too high concentration which provokes, after ions release in physiological fluids, the surface precipitation of AgCl in amounts not safe for human beings.

Silver ions have an oligodynamic effect, that means, depending on their concentration, together with the dimensions of cells with they interact, they could provide bacteriostatic, antiseptic or even cytotoxic activity [23-25], thus rising the importance of an accurate silver ions measuring on implants' surface [26].

The primary aim of this invention is to provide a process for the production of implantable prosthetic devices, temporary or definitive, which could come in contact with human beings for medium or prolonged times (for example months, years), able to release the adequate silver amounts to provide antibacterial action without causing systemic cytotoxicity.

At this purpose the main object of the present invention is a functionalization process with silver ions on glass and glass-ceramic surfaces for the production of implantable devices having an antibacterial action, as defined in the following claims.

The proposed process allows introducing silver ions solely into the very first surface layers of a glass or glass-ceramic material, shaped as a bulk material or as a coating, thus conferring the required antibacterial properties.

The prosthetic devices can thus have a body constituted by the above mentioned materials, or the main part formed by a substrate of any type of material, partially or totally provided with a coating of a glass or glass-ceramic material. Such device is afterwards subjected to the mentioned treatment, without the necessity for further manufacturing processes.

Materials to be used within the scope of the invention are preferably bioactive materials, and in particular bioactive glasses. Bioactive glasses are widely used as massive materials for small bone substitutions or as coatings on metallic or ceramic implants (artro-prostheses, dental implants). They are able to induce a real chemical bond with bone tissues because of their capability to interact with biological fluids [27] forming on their surface a layer of hydroxylapatite, which is the mineral constituent of calcified tissues. Bioactivity can be verified *in vitro* by the observation of hy-droxylapatite growing on a material's surface after soaking it in a simulated body fluid.

Nevertheless, the process can be applied generally to biocompatible glassy- or glass-ceramic materials, and not necessarily to bioactive ones.

The application of ion-exchange process, in conformity with the invention, to a biocompatible but not bioactive material, is in fact desirable and advantageous as it allows conferring antibacterial properties also to temporary or definitive devices which are expected to provide, depending on the implant site, besides the interaction with deep tissues, also an access to external environment through soft tissues (for example dental implant devices, orthopaedic devices for external fixation). In fact in the zone of transition from deep tissues to external ones is not necessary nor desirable to obtain a strong bond with tissues, but it becomes sufficient to guarantee biocompatibility properties and to control infections risks powerfully.

The only tie concerning the materials to be used in the invention ambit is represented by the necessary presence in such material of cations available to be exchanged with Ag⁺ cations. So, the employed materials contain modifiers ions, whose cations are susceptible to ion-exchange with silver ion, in particular from alkaline metals oxides (for example sodium or potassium) and alkaline-earth metals oxides.

It is also possible to use biocompatible glasses containing aluminium oxide. Employed glasses can be either based on silica systems or on phosphate ones.

The process object of the invention is carried out by the immersion of the device, or a part of its bulk, in an aqueous solution of a water-soluble silver salt. Preferably, the silver salt should be silver nitrate.

Water-soluble silver salt concentration can be varied in a wide range, but is generally lower than 0,5 M; the solution temperature is generally lower than 100°C, preferably between 80°C and 100°C; nevertheless, thermal treatment in high pressure conditions and at temperatures higher than 100°C can be also performed.

Time of treatment is not particularly critical and it is function of the temperature. Typically used process times are included between 15 and 240 minutes.

As the amount of introduced silver ions into the glassy material is as higher as higher is the amount of oxides containing exchangeable ions, is preferable to use glassy materials having a moderate content of exchangeable modifying ions; for example silica glasses, having sodium and/or potassium oxide content lower than 25% molar, are preferable.

The material obtained after applying the ion-exchange process, (here defined for conciseness as "exchanged" material) presents a thin surface layer concerned with silver ion functionalization, which typically has a thickness lower than 50 µm, better if lower than 1 µm. Exchanged material, after being immersed in simulated body fluid solutions or during time of implant in the body, can itself release silver ions, in a gradual and controlled manner during time, thus performing a local antibacterial action.

During some experimental tests, a bioactive glass composition, belonging to the system SiO₂-CaO-Na₂O, has been used; it has been synthesized by melting the starting compounds that are precursors of the constituent oxides of the glass and it is capable to exchange sodium ions with silver ions.

Exchanged glass has been characterized by means of morphological and compositional analyses (through electron microscopy - SEM - accompanied by EDS analysis), structural evaluations (X-Ray diffraction - XRD), spectroscopy (infra-red spectroscopy - FTIR_ATR) and *in vitro* tests both to verify exchanged material's bioactivity and to study silver ions release during time in simulated body fluid solutions. Such tests consisted in soaking the materials in a simulated body fluid, having the same composition of the blood plasma inorganic phase, and then observing both ion release trend and sample surface modifications during time.

After two months soaking in simulated body fluid, maintained at 37°C, the exchanged glass showed an unvaried bioactivity respect to untreated glass, as can be deduced by observing the growing of a thick layer of hydroxylapatite.

Silver ion release, evaluated during time by chemical analyses, of solutions that come in contact with the material, already occurs in the first hours after immersion, being this result perfectly compatible with antibacterial needs, which have to be performed especially during the first hours after the surgical operation. Moreover the release level is perfectly compatible with critical values for cells proliferation found out in literature and it prolongs for a sufficient time to maintain the antibacterial activity during the device employment.

Finally cytotoxicity tests on osteoblast-like cells have been performed, to verify biocompatibility of the modified material and bone cells. The same tests, for comparison purposes, have been also performed on samples of untreated glass and of glass exchanged in molten salts mixtures. The glass exchanged in aqueous solution has demonstrated compatibility towards cells comparable to the un-exchanged glass, while molten salts ion-exchanged glass resulted cytotoxic.

From these results we can deduce that the introduced silver amount by aqueous solution ion-exchange, and consequently released from the glass, has to be maintained under the limit of precipitation of AgCl to guarantee the non-toxicity of the surface.

On the base of these results, aqueous solutions ion-exchange process has been applied to another glass belonging to the same system, but selected for its lower surface reactivity respect to the previous one (the composition has been modifyed adding a small amount of Al₂O₃ that confer stability to the glass thus inhibiting its bioactivity).

In this way the exchange process applicability has been evaluated also for a non-bioactive glass, useful for applications where the formation of strong bonds with tissues is not necessarily required.

Also for this glass composition similar and in-depth biological characterizations have been performed (protein adsorption, adhesion and proliferation of fibroblasts) and also in this case the material resulted to be non-cytotoxic.

Further characteristics of the process relative to the invention are shown in the following example of application.

In the enclosed drawings:
- fig. 1 is a XRD plot relative to the glass used in the example of application and of a crystalline phase - wollastonite - obtained by thermal treatment at 950°C of the same glass;
- fig.2 is a SEM micrograph of a Ti₆Al₄V sample coated with SCNA glass, used in the example, and then subjected to aqueous solution ion-exchange process;
- fig.3 shows a graph in which Ag⁺ ions release data, collected by GFAAS in simulated body fluid, are compared for two glass samples series, one exchange by the process object of the present invention and the other by the exchange in molten salts mixture;
- fig.4 is a histogram representing the adhered (6 hours) and proliferated (24 hours) cells number, collected on the surface of a glass exchanged by the process object of the present invention (Ag-SCNA) in comparison with the untreated glass (SCNA) and the PS control; and
- fig.5 is composed by some SEM micrographs of cells adhered (6 hours) and proliferated (24 hours) on SCNA and Ag-SCNA samples surfaces.

### Example

Selected glass: SCNA

Molar composition: 57% SiO₂ - 34% CaO - 6% Na₂O - 3% Al₂O₃

Produced by melting at 1450-1550°C and pouring:
- on brass sheet to obtain massive samples;
- in water to obtain powders.

Massive samples have been subjected to annealing thermal treatment at 600°C for 10 hours.

The powder has been ground and sieved to a size between 30µm and 75µm to be sprayed by plasma-spray technique on a metallic substrate.

### Structural properties (XRD):

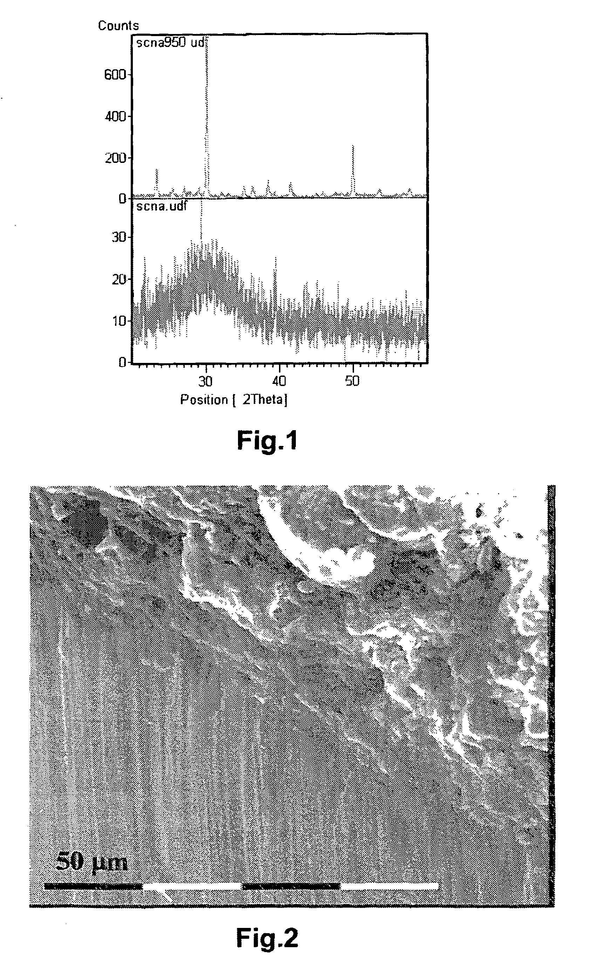

The glass obtained by melting and pouring is completely amorphous; with a thermal treatment at 950°C the crystalline phase named wollastonite (that is a bioactive ceramic) enucleates (fig.1)

With SCNA powder plasma spray coatings on Ti₆Al₄V (which is a Ti alloy usually used for dental implants and orthopaedic devices) tablets; coating thickness is about 100 µm.

### Ion-exchange in aqueous solution:

Both massive and coated samples have been subjected to the same ion-exchange process, being the conditions:
T = 100°C
[AgNO₃] = 0,05M
t = 45'

SEM observation of coated samples has shown that both the coating morphology and the substrate one remain unvaried after the aqueous solution ion-exchange process. It must be underlined that the metallic substrate does not reveal corrosion pits due to ion-exchange treatment (fig.2).

### Release tests (GFAAS) :

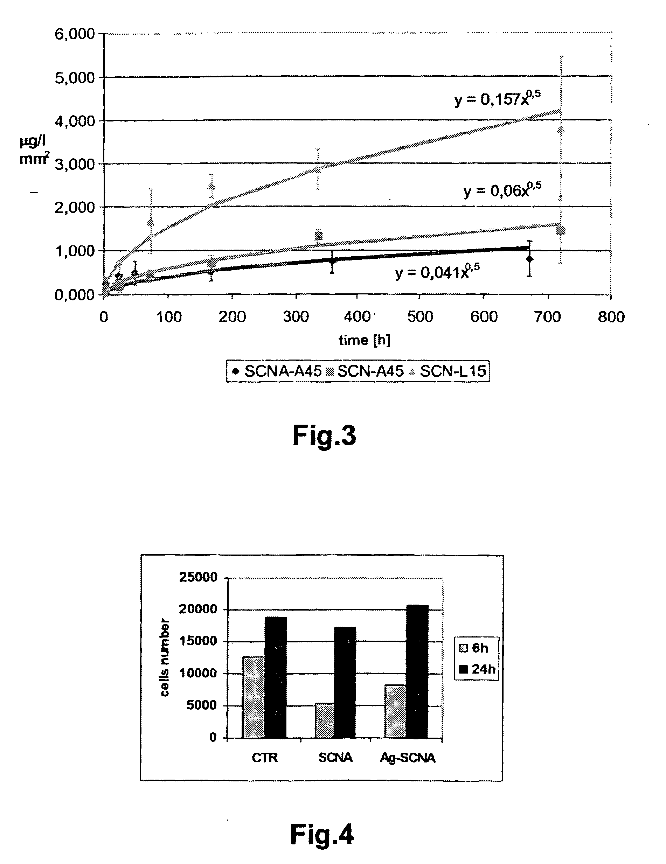

Release tests provide lower values of ion release respect to data collected on samples of the same glass exchanged in molten salts mixture or having a different glass composition (60% mol SiO₂ - 30% mol CaO - 10% mol NaO) (fig.3). XRD and EDS analyses performed on Ag-SCNA samples after 28 days soaking in simulated body fluid have not evidenced the presence of AgCl or other Ag salts on the sample surface. This point is very important as is known that AgCl has a cytotoxic behaviour.

### Biological properties (cell coltures):

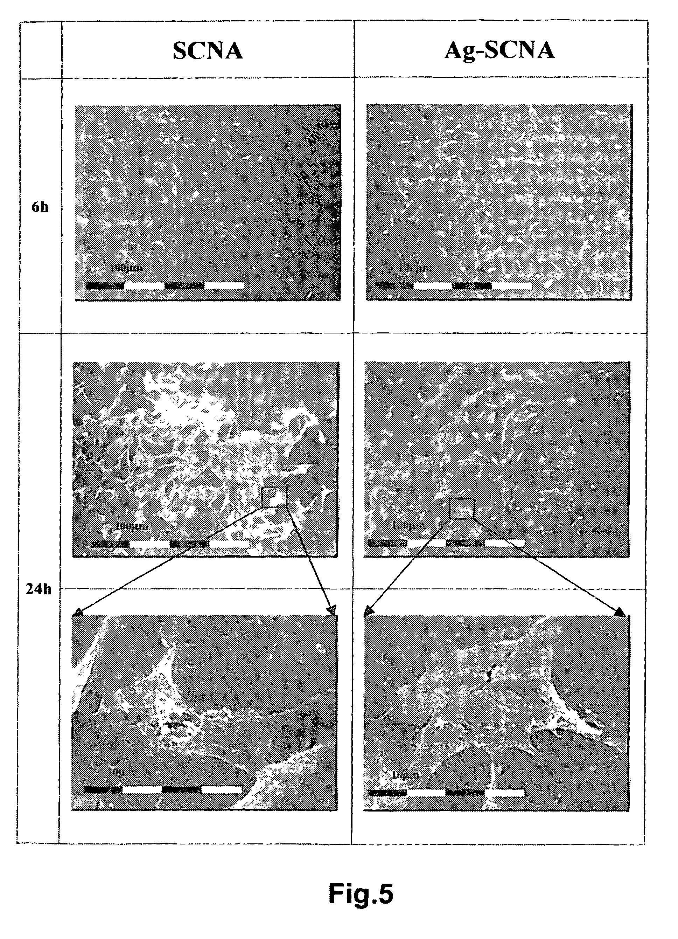

The glass behaviour has been non-cytotoxic (figg.4 and 5): in particular, by comparing micrographs in fig.5, it is clearly evident that Ag introduction in the glass does not significantly change neither the number, or the cells morphology.

The technique of the present invention, in respect of other processes described in literature [28-31] to obtain similar effects, shows some advantages, connected with many factors: the proposed technique is economic, easy to transfer on a large scale, versatile, and allows maintaining unchanged, except for the first surface layers, the structure and the chemical-physical characteristics of the glass. The presence of the antibacterial agent limited only to the surface, and not diffused in all the material's body, moreover allows to accurately tune the released silver, providing great material saving thus effectively maintaining its amount well below toxicity levels found out for silver ions when released in the human body in excessive doses. Moreover, silver ions release is limited to the first hours after the implant, when it is strictly recommended to guarantee the antibacterial action. Finally, the functionalization by the introduction of silver ions can be limited to small areas of a prosthetic device, and not necessarily applied to the all surface, thus allowing realizing a multi-functional device. Such prosthetic devices, temporary or definitive, can be used in all those applications that can be realized with the before mentioned materials and that require an antiseptic action after their implant. Applications in the orthopaedic surgery, maxillofacial surgery and devices for the controlled release in drug delivery systems are to take into consideration.

### Bibliography

1. M.Confalonieri, E.Damonti: Microbiological patterns in osteo-articular prosthetic infections. GIMMOC 2003; VII [1] : 21-26.
2. RO Becker, JA Spadaro: Treatment of orthopedic infections with electrically generated ions. J Bone Joint Surg 1978; 60[7]: 871-881.
3. DA Webster, JA Spadaro, RO Becker, S.Kramer: Silver Anode Treatment of Chronic Osteomyelitis. Clinical Orthopaedics and Related Research 1981; 161: 105-114.
4. QL Feng, J.Wu, GQ Chen, FZ Cui, TN Kim, JO Kim: A mechanistic study of the antibacterial effect of silver ions on Escherichia coli and Staphylococcus aureus. J Biomed Mater Res 2000; 52: 662-668.
5. TN Kim, QL Feng, JO Kim, J.Wu, H.Wang, GC Chen, FZ Cui: Antimicrobial effects of metal ions (Ag+, Cu2+, Zn2+) in hydroxyapatite. J Mat Sci: Mater Med 1998; 9: 129-134.
6. C.Chu, AT McManus, BA Pruitt Jr, AD Mason Jr: Therapeutics Effects of Silver Nylon Dressings with Weak Direct Current on Pseudomonas Aeruginosa-Infected Burn Wounds. The Journal of Trauma 1988; 28[10]: 1488-1492.
7. K.Yoshida, M.Tanagawa, M.Atsuta: Characterization and inhibitory effect of antibacterial dental resin composites incorporating silver-supported materials. J Biomed Mater Res 1999; 47: 516-522.
8. L.Balogh, DR Swanson, DA Tomalia, GL Hagnauer, AT McManus: Dendrimer-Silver Complexes and Nanocomposites as Antimicrobial Agents. Nano Letters 2001; 1[1]: 18-21.
9. DP Dowling, K.Donnelly, ML McConnell, R.Eloy, MN Arnaud : Deposition of antibacterial silver coatings on polymeric substrates. Thin Solid Films 2001; 398-399: 602-606.
10. S.Saint, JG Elmore, SD Sullivan, SS Emerson, TD Koepsell: The Efficacy of Silver Alloy-coated Urinary Catheters in Preventing Urinary Tract Infection: A Meta-Analysis. Am J Med 1998; 105: 236-241.
11. JE Gray, PR Norton, R.Alnouno, MA Valvano, K. Griffiths: Biological efficacy of electroless-deposited silver on plasma activated polyurethane. Biomaterials 2003; 24: 2759-2765.
12. K.Yoshida, M.Tanagawa, S.Matsumoto, T.Yamada, M.Atsuta: Antibacterial activity of resin composites with silver-containing materials. Eur J Oral Sci 1999; 107: 290-296.
13. G.Muller, Y.Winkler, A.Kramer: Antibacterial activity and endotoxin-binding capacity of Actisorb® Silver 220. J Hosp Infect 2003; 53: 211-214.
14. A.Massè, E.Rizzo, G.Fortina, GL Molinari, A. Bruno, M.Cannas: Prevenzione delle infezioni su biomateriali at-traverso il rivestimento con argento - valutazione in vitro dell'adesività batterica. Riv It Biol Med 1996; 16: 1-4.
15. MA Wassall, M.Santin, C.Isalberti, M.Cannas, SP Denyer: Adhesion of bacteria to stainless steel and silver-coated orthopaedics external fixation pins. J Biomed Mater Res 1997; 36: 325-330.
16. M.Bosetti, A.Massè, E.Tobin, M.Cannas: Silver coated materials for external fixation devices: in vitro biocompatibility and genotoxicity. Biomaterials 2002; 23: 887 - 892.
17. A. Massè, A.Bruno, M.Bosetti, A.Biasibetti, M. Cannas, P.Gallinaro: Prevention of pin track infection in external fixation with silver coated pins: clinical and microbiological results. J Biomed Mater Res (Appl Biomater) 2002; 53: 600-604.
18. QL Feng, FZ Cui, TN Kim, JW Kim: Ag-substituted hydroxyapatite coatings with both antibacterial effects and biocompatibility. J Mater Sci: Lett 1999; 18: 559-561.
19. AG Avent, CN Carpenter, JD Smith, DM Healy, T. Gilchrist: The dissolution of silver-sodium-calcium-phosphate glasses for the control of urinary tract infections. J Non-Crys Sol 2003; 328: 31-39.
20. T.Kasuga, H.Kume, Y.Abe: Porous glass-ceramics with bacteriostatic properties in silver-containing titanium-phosphates: control of release of silver ions from glass ceramics into aqueous solution. J Am Ceram Soc 1997; 80 [3]: 777-780.
21. Y.Inque, Y.Kanzaki: The mechanism of antibacterial activity of silver-loaded zeolite. Journal of Inorganic Biochemistry 1997; 67: 1-4.
22. M.Bellantone, NJ Coleman, LL Hench: Bacteriostatic action of a novel four-component bioactive glass. J Biomed Mater Res 2000; 51: 484-490.
23. SS Djoric, RE Burrel: Behavior of silver in physiological solutions. J Electrochem Soc 1998; 145[5]: 1426-1430.
24. TJ Berger, JA Spadaro, SE Chapin, RO Becker: Electrically generated silver ions: quantitative effects on bacterial and mammalian cells. Antimicrob Agents Chemother 1976; 9[2]: 357-358.
25. RJ Williams, PJ Doherty, DG Vince, GJ Grashoff, DF Williams: The biocompatibility of Silver. Critical Reviews in Biocompatibility 1989; 5[3]
26. CN Kraft, M.Hansis, S.Arens, MD Menger, B. Vollmar: Striated muscle microvascular response to silver implants: a comparative in vivo study with titanium and stainless steel. J Biomed Mater Res 2000; 49: 192-199.
27. LL Hench: Bioceramics, J Am Ceram Soc 81[7] (1998) 1705-28
28. M.Kawashita, S.Tsuneyama, F.Miyaji, T.Kokubo, H.Kozuka, K.Yamamoto: Antibacterial silver-containing silica glass prepared by sol-gel method. Biomaterials 2000; 21: 393-398.
29. HJ Jeon, SC Yi, SG Oh: Preparation and antibacterial effects of Ag-Si02 thin films by sol-gel method. Biomaterials 2003; 24: 4921-4928.
30. M.Kawashita, S.Toda, HM Kim, T.Kokubo, N.Masuda: Preparation of antibacterial silver-doped silica glass microspheres. J Biomed Mater Res 2003; 66A: 266-274.
31. M.Bellantone, NJ Coleman, LL Hench: A novel sol-gel derived glass featuring antibacterial properties. Bioceramics. Key Engineering Materials 2001; 192-195: 597-600.
32. S.Di Nunzio, C. Vitale Brovarone, S.Spriano, D. Milanese, E. Vernè, V. Bergo, G.Maina, P.Spinelli: Silver containing bioactive glasses prepared by molten salt ion-exchange. J Eur Cer Soc 24(2004)2699-2705.

## Claims

1. Process for the preparation of a prosthetic device or implant, adapted to release silver ions in the implant site, thus providing an antibacterial but non-cytotoxic action, wherein said device or implant has a body constituted by a glassy or glass-ceramic material or is provided with a partial or total coating of said materials, **characterized in that** said materials contain modifying oxide of metals which can be exchanged with silver ions, selected from alkaline and alkaline earth metals, the process comprising subjecting said device or implant to an ion exchange process in a silver containing aqueous solution.

2. Process according to claim 1, **characterized in that** said ion-exchange process is performed by the device immersion in a soluble silver salt aqueous solution.

3. Process according to claims 1 or 2, **characterized in that** said solution contains silver nitrate in a concentration smaller than 0,5 M.

4. Process according to any of claims 1 to 3, **characterized in that** the ion-exchange process is performed at temperature lower than 100°C, preferably between 80°C and 100°C for periods from 15' to 240'.

5. Process according to any of claims 1 to 4, **characterized in that** said material is a bioactive glass.

6. Process according to any of claims 1 to 5, **characterized in that** said material is a glass containing silica, calcium oxide, sodium oxide and optionally aluminium oxide.

7. Process according to any of the previous claims, **characterized in that** said material contains an amount of sodium oxide not exceeding 25% molar.

## Patentansprüche

1. Verfahren zur Herstellung einer Prothese oder eines Implantats, die/das geeignet ist, im Implantationsbereich Silberionen freizusetzen, was somit eine antibakterielle, aber nicht-zytotoxische Wirkung bereitstellt, wobei die Prothese oder das Implantat einen Körper aufweist, der aus einem glasartigen oder Glaskeramik-Material gebildet wird oder mit einer teilweisen oder vollständigen Beschichtung des Materials versehen ist, **dadurch gekennzeichnet, dass** die Materialien modifizierende Metalloxide enthalten, die gegen Silberionen ausgetauscht werden können, die aus Alkali- und Erdalkalimetallen ausgewählt sind, wobei das Verfahren umfasst, die Prothese oder das Implantat einem Ionenaustauschverfahren in einer Silber-enthaltenden wässrigen Lösung zu unterziehen.

2. Verfahren nach Anspruch 1, **dadurch gekennzeichnet, dass** das Ionenaustauschverfahren durchgeführt wird, indem die Prothese in eine wässrige Lösung eines löslichen Silbersalzes eingetaucht wird.

3. Verfahren nach den Ansprüchen 1 oder 2, **dadurch gekennzeichnet, dass** die Lösung Silbernitrat in eine Konzentration von weniger als 0,5 M enthält.

4. Verfahren nach einem der Ansprüche 1 bis 3, **dadurch gekennzeichnet, dass** das Ionenaustauschverfahren bei einer Temperatur von weniger als 100°C, bevorzugt zwischen 80°C und 100°C, für Zeitspannen von 15' bis 240' durchgeführt wird.

5. Verfahren nach einem der Ansprüche 1 bis 4, **dadurch gekennzeichnet, dass** das Material ein bioaktives Glas ist.

6. Verfahren nach einem der Ansprüche 1 bis 5, **dadurch gekennzeichnet, dass** das Material ein Glas ist, das Siliciumdioxid, Calciumoxid, Natriumoxid und gegebenenfalls Aluminiumoxid enthält.

7. Verfahren nach einem der vorhergehenden Ansprüche, **dadurch gekennzeichnet, dass** das Material eine Menge an Natriumoxid von höchstens 25 Mol-% enthält.

## Revendications

1. Procédé pour la préparation d'un dispositif prothétique ou implant, adapté pour libérer des ions argent dans le site d'implantation, en réalisant ainsi une réaction antibactérienne mais non cytotoxique, où ledit dispositif ou implant a un corps constitué par un matériau vitreux ou vitrocéramique ou est muni d'un revêtement partiel ou total desdits matériaux, **caractérisé en ce que** lesdits matériaux contiennent un oxyde modifiicateur de métaux qui peuvent être soumis à un échange avec des ions argent, choisis parmi les métaux alcalins et alcalino-terreux, le procédé comprenant l'exposition dudit dispositif ou implant à un procédé d'échange d'ions dans une solution aqueuse contenant de l'argent.

2. Procédé selon la revendication 1 **caractérisé en ce que** ledit procédé d'échange d'ions est mis en oeuvre par l'immersion du dispositif dans une solution aqueuse de sel d'argent soluble.

3. Procédé selon la revendication 1 ou 2 **caractérisé en ce que** ladite solution contient du nitrate d'argent en une concentration inférieure à 0,5 M.

4. Procédé selon l'une quelconque des revendications 1 à 3 **caractérisé en ce que** le procédé d'échange d'ions est mis en oeuvre à une température inférieure à 100°C, de préférence entre 80°C et 100°C pendant des durées de 15' à 240'.

5. Procédé selon l'une quelconque des revendications 1 à 4 **caractérisé en ce que** ledit matériau est un verre bioactif.

6. Procédé selon l'une quelconque des revendications 1 à 5 **caractérisé en ce que** ledit matériau est un verre contenant de la silice, de l'oxyde de calcium, de l'oxyde de sodium et éventuellement de l'oxyde d'aluminium.

7. Procédé selon l'une quelconque des revendications précédentes **caractérisé en ce que** ledit matériau contient une quantité d'oxyde de sodium ne dépassant pas 25 % molaires.
